# EUROPEAN PATENT SPECIFICATION

(11) **EP 2 319 573 B1**
(45) Date of publication and mention of the grant of the patent: **04.07.2012**
(21) Application number: 09425442.2
(22) Date of filing: 04.11.2009
(51) Int. Cl.: A61M 16/10, A61M 16/20, F16K 3/26, F16K 11/07, A61M 16/08

(54) **Respiratory device for medication delivery.**
Atemgerät zur Abgabe von Arzneimitteln
Dispositif respiratoire pour administration de médicaments

(43) Date of publication of application: 11.05.2011
(73) Proprietor: Covidien AG, 8212 Neuhausen am Rheinfall (CH)
(72) Inventor: Resca, Daniele, 41038 San Felice sul Panaro (Modena) (IT); Zucchi, Giuseppe, 41039 San Possidonio (Modena) (IT); Romagnoli, Elisa, 44042 Cento (Ferrara) (IT)
(74) Representative: Lanzoni, Luciano

(56) References cited:
- EP-A1- 1 192 968
- WO-A1-2009/149290
- DE-U1- 29 805 569
- US-A1- 2004 123 974
- US-A1- 2008 210 242

## Description

The present invention relates to a respiratory device capable of conditioning respiratory gases in combination with a path intended for medication delivery.

Conditioning is intended as heating and humidification and/or filtration.

In particular, the present invention is suitable for use connected to equipment for mechanical ventilation.

During mechanical ventilation, the respiratory gases fed from the ventilator to the patient shall have adequate temperature level and moisture content because the patient has bypassed upper airways due to tracheal intubation. Heat and humidity can be added to respiratory gases through active humidifiers. In alternative, prevention of heat and moisture loss from airways can be achieved by means of devices called HME (Heat and Moisture Exchanger) or artificial noses able to trap heat and humidity during exhalation and returning good portion of the same to the patient in inhalation phase. In addition, respiratory gases can be filtered through suitable means called antibacterial/antiviral filters.

In the present application, "respiratory gases" is intended to identify either air or other gas mixture indifferently.

These respiratory devices are therefore fitted with suitable members able to act as heat and moisture exchangers (HME) and filters.

Frequently, a patient connected to a mechanical ventilator also requires aerosol therapy.

In this case, a nebulised medication is dispensed into the respiratory gases streamline.

It is important that the flow of gases conveying the nebulised medication does not pass through said member (the HME and/or filter) because the medication would otherwise be partially absorbed by the member itself. This would result in the waste of medication, inefficiency of treatment and also lead to deterioration and clogging of the member (exchanger/filtering element).

To overcome this drawback, some passive humidification devices currently available on the market comprise an HME element positioned along the main channel, and an alternative path excluding the heat and moisture exchanger bypassing it.

Inside, these devices have a selector that directs the air flow only towards the heat and moisture exchanger, when the humidification mode is required, and switches the flow of gases towards the bypass when the alternative medication mode is selected.

A drawback pointed out is that the diverter must be switched manually by the operator as shown in document EP 1192968. Selecting from one operating position to another is not always easy especially if the device is being operated by one person alone as is often the case in hospitals or when patients are receiving treatment at home. During operation of the diverter, the device must be kept in position by the operator ensuring that it does not detach from the portion of respiratory circuit that conveys respiratory gases from the ventilator to the patient.

The devices which are currently commercially available have at least three components to be handled during the switching phase and this factor clearly complicates matters when a person has to operate the device alone.

Furthermore, the devices currently commercially available need to be switched manually from humidification mode to aerosol mode before to apply the medication and the possibility of human error in forgetting to perform this manoeuvre is present. At the same time, once the medication treatment is completed, the operator shall remember to switch back from aerosol to humidification mode.

The technical purpose of the present invention is to overcome the drawbacks described above by providing a respiratory device capable of exchanging heat and moisture and/or filtering respiratory gases coupled with an alternative path for medication delivery. The present invention is described in claim 1 and is aimed to provide a respiratory device capable of exchanging heat and moisture and/or filtering respiratory gases in combination with an alternative secondary path for aerosol delivery which is easy to operate for one person alone and which allows automatic switching from humidification to aerosol delivery mode upon insertion of an aerosol delivery device. This automatic system enhances safety of the device eliminating human error.

Another purpose of the invention is to provide a respiratory device capable of exchanging heat and moisture and/or filtering respiratory gases in combination with an alternative secondary path for aerosol delivery which is simple and inexpensive to manufacture.

The technical purpose and aims specified are substantially achieved by a respiratory device capable of exchanging heat and moisture and/or filtering respiratory gases in combination with a secondary path for aerosol delivery, comprising the technical characteristics described in one or more of the claims below.

Further features and advantages of the present invention will become apparent from the description below of a preferred embodiment shown in the annexed drawings, given purely by way of a nonlimiting example, in which:
- Figure 1 is a perspective view of a first embodiment of the device according to the present invention;
- Figure 2 is an exploded view of the device shown in Figure 1;
- Figure 3 is a cross-section of the device shown in Figure 1 in a first operating position;
- Figure 4 is a cross-section of the device shown in Figure 1 in a second operating position;
- Figure 5 is a perspective view of a second embodiment of the device according to the present invention;
- Figure 6 is an exploded view of the device shown in Figure 5;
- Figure 7 is a cross-section of the device shown in Figure 5 in a first operating position;
- Figure 8 is a cross-section of the device shown in Figure 5 in a second operating position;
- Figure 9 is an enlarged view of a component of the device shown in Figure 2;
- Figure 10 is an enlarged view of a component of the device shown in Figure 6.

In the drawings, the numeral 1 denotes in its entirety a respiratory device capable of exchanging heat and moisture and/or filtering respiratory gases in combination with a secondary conduit dedicated to convey aerosol.

The respiratory device 1 is positioned along the breathing circuit between Y-piece and patient interface (endotracheal or tracheostomy tube) conveying ventilation gases to a patient.

In this way, a generic respiratory circuit is divided into a first section M upstream, located between the ventilator and the device, and a second section P downstream, located between the device and the patient.

The device is connected to an aerosol delivery device enabling to perform aerosol therapy.

As described below, the present invention comprises two alternative paths, one designed exclusively for the passage of gases for normal respiration, and the other exclusively for the passage of gases and nebulised medication.

The device 1 comprises a primary pathway 20 defined by an inlet channel A, a first intermediate channel 2, an housing 3 and an outlet channel B.

The housing 3 contains a conditioning member 40 which in the preferred embodiments shown comprises a heat and moisture exchanger element 4 and a filtering element 5.

According to different embodiments not shown of the present invention, the conditioning member 40 comprises either a heat and moisture exchanger or a filtering element.

When gases travel along primary pathway 20, gases flow through the conditioning member 40.

The device 1 comprises, in addition, a secondary pathway 21 defined, in the direction of the gases flow F from ventilator side to patient side respectively, by the inlet channel A, a second intermediate channel 6 and the outlet channel B. With reference to any of the drawings, flow direction F is oriented from the section M of the main tube connected to the ventilator proceeding towards the section P of the main tube connected to the patient.

Channels A and B are parts in common between pathway 20 and 21.

Both the primary pathway 20 and the secondary pathway 21 are selectively and alternately in fluid communication with the main respiratory tube when the device 1 is incorporated in the respiratory circuit.

The device 1 comprises an automatic valve 10 capable of selectively opening the second intermediate channel 6 and excluding the first intermediate channel 2 when required.

In other words, during normal respiration the valve 10 allows the flow of gases to pass through the primary pathway 20 only, while during aerosol therapy the valve 10 allows the flow of gases and nebulised medication to pass through the secondary pathway 21 only.

Advantageously, the automatic valve 10 is located in correspondence of inlet channel A where pathway 20 and pathway 21 differentiate one from the other. In other words, the automatic valve 10 is located, with reference to the pathway 20, between the inlet channel A and the first intermediate channel 2, and with reference to the pathway 21, between the inlet channel A and the second intermediate channel 6. The device 1 has, in the inlet channel A, a single tube which then divides into the first intermediate channel 2 and the second intermediate channel 6. Downstream the housing 3, the separated primary pathway 20 and the secondary pathway 21 join to form a single tube which defines the outlet channel B of the device 1.

The valve 10 is automatic and is actuated by simply coupling the valve 10 itself to an external element 11. The external element 11 can be for example a nebulizer containing a therapeutic substance or medication, to be nebulised.

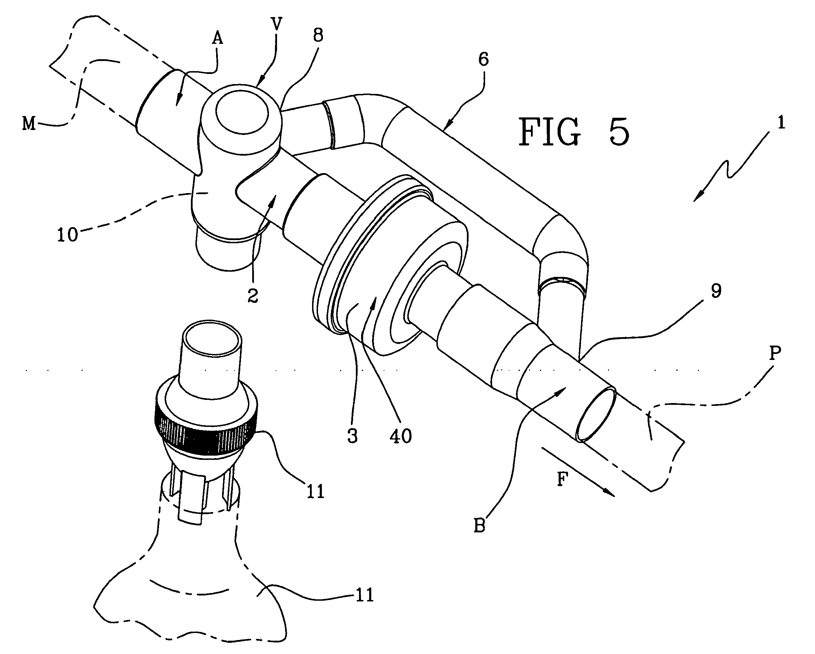

Alternatively, the external element 11 can be an adapter with means for dispensing drugs contained in a metered dose inhaler (MDI), not illustrated. In Figure 5 the reference numeral 11 generally indicates both a nebulizer, shown with broken lines, and MDI adapter.

The valve 10 is movable between a first operating position where only the primary pathway 20 is active and a second operating position where only the secondary pathway 21 is active.

In other words, in the first operating position the valve 10 is in fluid communication with the first intermediate channel 2 while in the second operating position the valve 10 is in fluid communication with the second intermediate channel 6.

In the present description the expression "in fluid communication" referred to different parts means that there is a fluid connection between said parts.

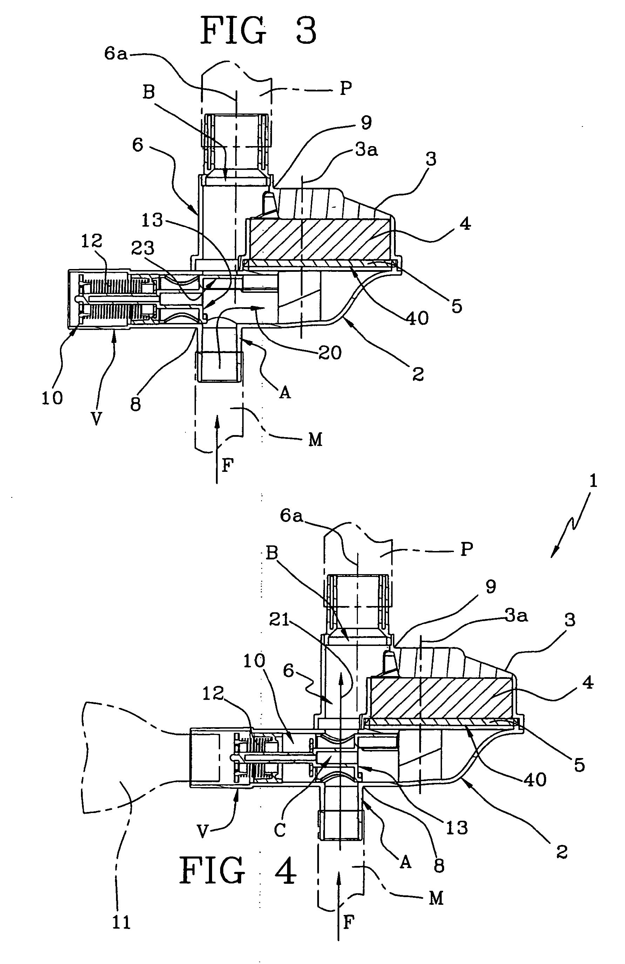

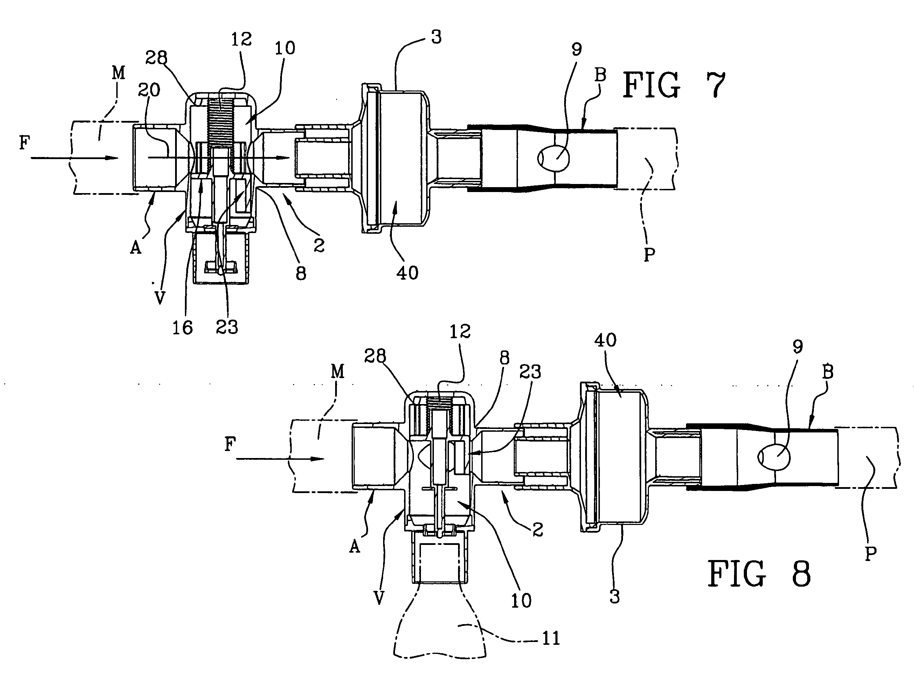

After coupling of the valve 10 to an external element 11, the valve 10 itself moves from the first operating position (Figures 3 and 7) to the second operating position (Figures 4 and 8); the removal of the element 11 deactivates the valve 10 and returns it to the first operating position (Figures 3 and 7).

The movement of the valve 10 from the second position back to the first position is facilitated by opposing means 12. This opposing means 12 serves also to keep the valve 10 in the first position when no medication delivery is performed.

The opposing means 12 comprises a helical spring which is partially compressed and loaded when the valve 10 is in the rest position and is further compressed and loaded when the element 11 is coupled to the valve 10 thereby moving the valve 10 into the second operating position.

When the element 11 is removed, on the other hand, the spring 12, compressed and loaded, acts on the valve 10 to return it to the rest position, pushing it into the first operating position (Figures 3 and 7).

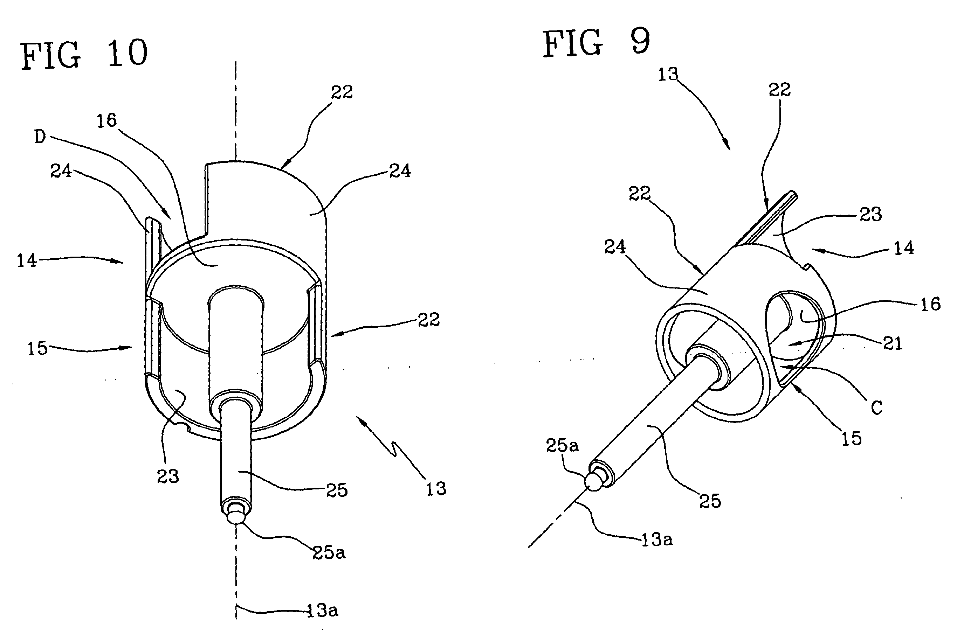

Figures 9 and 10 show that the automatic valve 10 is a two-way valve and comprises a main body 13 divided into a first level 14 and a second level 15 by a central septum 16.

In a preferred embodiment, the main body 13 has a cylindrical form extending along the longitudinal axis 13a.

The first level 14 defines the pathway 20 for the gases flow only, while the second level 15 defines the pathway 21 for the flow of gases and nebulised medication.

In particular, both the first level 14 and the second level 15 comprise opening/closing means 22 which defines the pathway 20 for the flow of gases and the pathway 21 for the flow of gases and nebulised medication, closing alternatively the second intermediate channel 6 and the first intermediate channel 2.

Preferably, the opening/closing means 22 comprises at least a finger nail 23 and at least a wall 24. The term 'finger nail' in this description is to be understood as a portion of the cylindrical wall as clearly shown in the drawing.

During ventilation, the flow of gases coming from ventilator enters the inlet channel A of the device 1, passes through the first level 14 of the valve 10, flows into the first intermediate channel 2 passing through the conditioning member 40, then leaves through the outlet channel B of the device 1 and passes onwards to the patient. The respiratory gases exhaled during normal respiration follows the same path but in the opposite direction.

During aerosol therapy by means of the element 11 the valve 10 is actuated and the flow of respiratory gases mixed with nebulised medication passes through the second intermediate channel 6 up to the outlet channel B of the device 1 and onwards to the patient.

The main body 13 of the valve 10 comprises in addition a pin 25 which extends along the axis 13a from the septum 16 through the side of the second level 15. The pin 25 has a first end connected to the septum 16 and a second free end 25a opposite to the first end. The free end 25a is connected to a pusher disc 26, parallel to the septum 16, which acts as a support surface for the external element 11. In other words, the external element 11 presses against the pusher disc 26 to move the valve 10 from the first operating position to the second operating position.

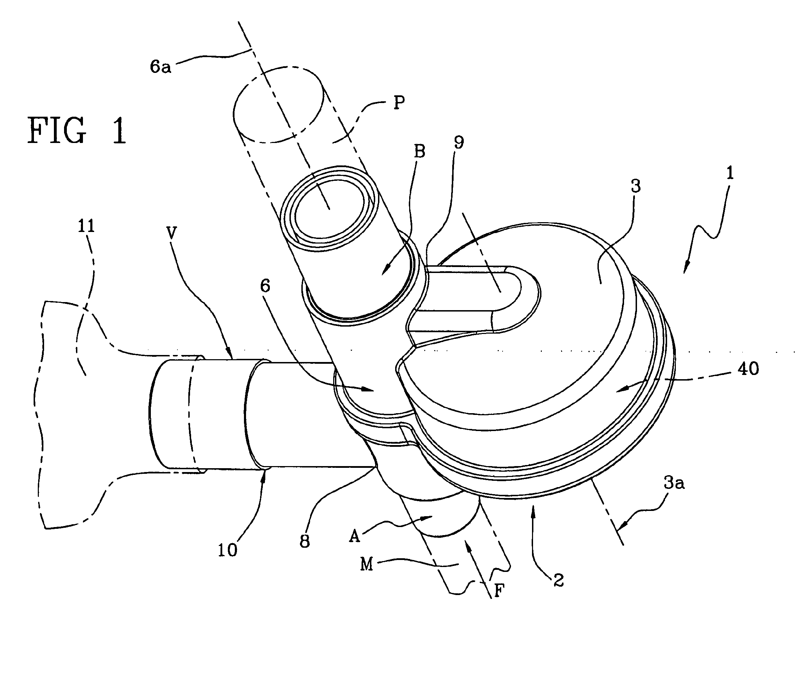

Figures 1 to 4 and also Figure 9 show a first embodiment of the device 1 according to the present invention which will now be described.

In the first embodiment of the present invention, the device 1 has a substantially compact configuration. The valve 10 is aligned with a first section of the first intermediate channel 2 and is at right angles to the second intermediate channel 6. The second intermediate channel 6 has a longitudinal axis 6a parallel to the axis 3a of the housing 3 of the heat and moisture exchanger element 4 and/or filter element 5 (i.e. the conditioning member 40).

Figure 9 shows a detailed view of the main body 13 of the valve 10 according to the first embodiment of the device 1, in which the first level 14 has the opening/closing means 22 comprising the finger nail 23. When the automatic valve 10 is in the first operating position, the nail 23 defines an inside surface for the flow of gases along the primary pathway 20.

In the first operating position of the valve 10 therefore, the nail 23 of the first level 14 is designed to close the second intermediate channel 6.

On the second level 15, the opening/closing means 22 has at least one wall 24 which extends at right angles to the septum 16. This wall 24 has a transversal channel C, visible in Figure 9, for the passage of the flow of gases and nebulised medication when the valve 10 is in the second operating position.

In this first embodiment, when the automatic valve 10 is in the second operating position, the septum 16 closes the first intermediate channel 2 to enable the passage of the flow of gases and nebulised medication along the second intermediate channel 6, through the secondary pathway 21.

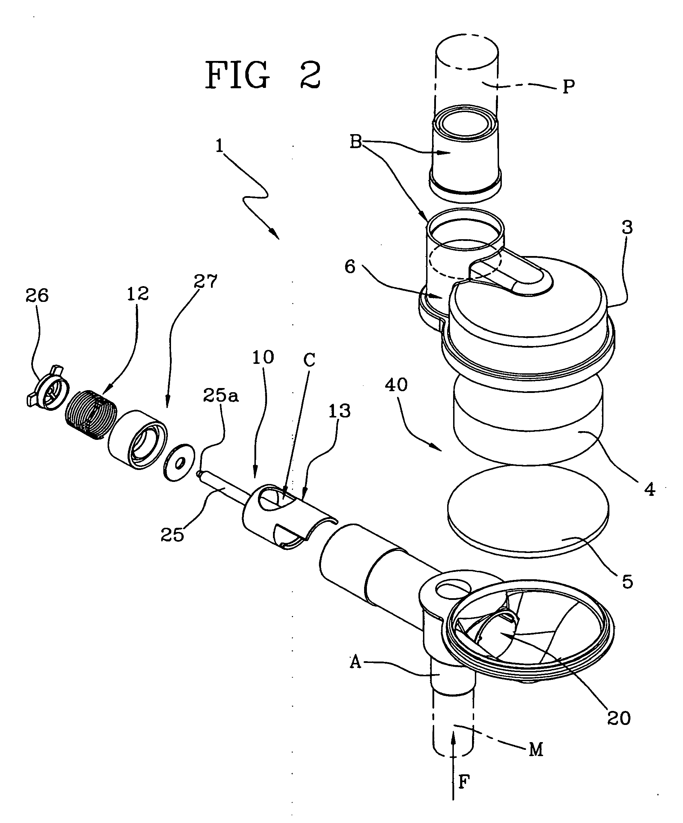

Between the septum 16 and the pusher disc 26 there is an opposing means 12 (Figure 2).

The opposing means 12, suitably housed inside containing means 27, preferably comprises a spring pushing the valve 10 from the second operating position to the first operating position.

During use, when the device 1 is used for normal respiration, the valve 10 is in the first operating position (Figure 3).

In this position, the second level 15 of the main body 13 is completely housed in the containing seat V of the valve 10. The first level 14, on the other hand, is in fluid communication with the inlet channel A of the device 1. The nail 23 closes access to the second intermediate channel 6 so that the flow of gases can only flow towards the first intermediate channel 2 through the primary pathway 20 and through the conditioning member 40, then leaving the housing 3 through the outlet channel B of the device 1 into which also the second intermediate channel 6 is inserted.

When the device 1 is in the aerosol therapy mode, as shown in Figure 4, the external element 11 dispensing the nebulised medication or more generically a therapeutic substance, is coupled to the valve 10. This coupling moves the valve 10 into the second operating position. In this position, the first level 14 moves forward inside the first intermediate channel 2 and the septum 16 thereby closes off access to the first intermediate channel 2 itself.

The second level 15 moving forward, leaves the containing seat V of the valve 10 and moves into fluid communication with the inlet channel A of the device 1. The pathway 21 passes through a transversal channel C to put the inlet channel A into fluid communication with the second channel 6. Figures 5 to 8 and also Figure 10 show a second embodiment of the device 1 according to the present invention.

This second embodiment has a double-Y shaped configuration where the inlet channel A of the device 1 divides, at the first joint 8 upstream the conditioning member 40, into a primary pathway 20 and a secondary pathway 21.

The primary pathway 20 comprises the first intermediate channel 2 and the housing 3 of the member 40 placed in the middle of the channel 2 itself.

Thus, the first intermediate channel 2 and the second intermediate channel 6 form a first Y-shape at the first joint 8 upstream.

The two intermediate channels 2 and 6 join together again downstream the conditioning member 40, to form a second Y-shape, at the second joint 9 where the outlet channel B of the device 1 starts.

The valve 10 is at right angles to the containing plane of the two intermediate channels 2 and 6. According to the second embodiment of the invention, the opening/closing means 22 comprises on the first level 14, at least one wall 24 designed to close the second intermediate channel 6, and to define the primary pathway 20 for the flow of gases along the first intermediate channel 2 only, when the valve 10 is in its first operating position (Figures 7 and 10).

In a preferred embodiment of the main body 13, there are two walls 24 (Figure 10) between which there is a transversal channel D which defines the primary pathway 20 for the flow of respiratory gases.

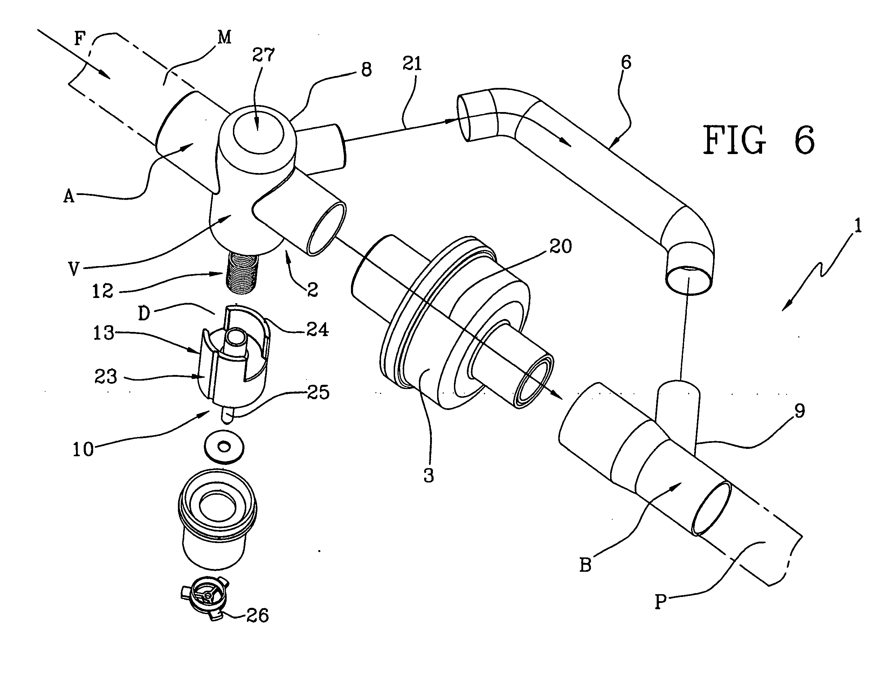

The opening/closing means 22 comprises, on the second level 15, a nail 23 designed to close the first intermediate channel 2, when the automatic valve 10 is in the second operating position, as shown in Figure 8. This defines a secondary pathway 21, indicated in Figure 6, for the flow of gases and medication which is diverted towards the second intermediate channel 6 only.

This second embodiment also has opposing means, a spring 12, suitably housed inside containing means 27, which tends to return the valve 10 from the second operating position to the first operating position (Figure 6).

In particular, in this case the opposing means 12 is positioned between the septum 16 and a bottom wall 28 of the space V housing the valve 10 on the side of the first level 14.

In this case also, when the valve 10 is in the first operating position, the first level 14 is in fluid communication with the inlet channel A of the device 1 and with the first intermediate channel 2. During aerosol therapy, the external element 11 is coupled to the valve 10. The valve 10 is pushed into the second operating position, the spring 12 is compressed and the second level 15 of the main body 13 of the valve 10 is activated. In this configuration, the second level 15 is in fluid communication with the inlet channel A of the device 1 and with the second intermediate channel 6.

The present invention has notable advantages and achieves the purposes described above.

The presence of an automatic valve prevents any risk of human error in positioning the flow diverter when switching backwards and forwards between the normal respiration and the aerosol therapy modes.

The channel for the required operating mode is selected automatically when the valve is coupled to the aerosol/medication dispensing element. When no aerosol/medication dispensing element is present, the operator can be certain that the device is set for the normal respiration mode.

The device is easy for a single operator to use and provides a clear, precise visual indication of the device operating mode currently selected.

## Claims

1. A respiratory device for conditioning respiratory gases, comprising
a primary pathway (20) and
a housing (3) placed in fluid communication with the primary pathway (20) and containing a conditioning member (40),
a secondary pathway (21) for medication delivery connected to the primary pathway (20) both upstream and downstream the housing (3) referring to a direction (F) of the flow of gases, an automatic valve (10) capable of selectively activating the secondary pathway (21) and excluding the primary pathway (20); **characterised in that** said valve (10) is slidingly movable between a first operating position where only the primary pathway (20) is active and a second operating position where only the secondary pathway (21) is active; the automatic valve (10) being activated by a pushing action when an external element (11) is coupled to it, so that it moves into the second operating position, and is deactivated by removing the external element (11) so that it returns to the first operating position.

2. The respiratory device according to claim 1, in which said primary and secondary pathways (20, 21) have a common inlet channel (A), **characterized in that** said automatic valve (10) is positioned in correspondence of said inlet channel (A).

3. The respiratory device according to any of claims 1 or 2, **characterised in that** it comprises opposing means (12) designed to hold or return the valve (10) in the first operating position.

4. The respiratory device according to claim 3, **characterised in that** the opposing means (12) comprises a helical spring, said spring being compressed and loaded by the valve (10) when the external element (11) is coupled to the valve (10) which is thereby pushed into the second operating position and which is extended in the rest position when the valve (10) is in the first operating position.

5. The respiratory device according to any of claims 1 to 4, **characterised in that** the automatic valve (10) is a two-way valve and comprises a main body (13) divided into a first level (14) and a second level (15) by a central septum (16).

6. The respiratory device according to claim 5, **characterised in that** the first level (14) and the second level (15) comprise opening/closing means (22) which let the gases flowing through the primary pathway (20) on the first level (14) and the gases mixed with a nebulised medication through the secondary pathway (21) on the second level (15), closing respectively and alternatively the secondary pathway (21) and the primary pathway (20).

7. The respiratory device according to claim 7, 5 or 6, **characterised in that** said main body (13) comprises a pin (25) which extends axially and centrally from the septum (16) towards the second level (15).

8. The respiratory device according to claim 7, **characterised in that** the main body (13) comprises a pusher disc (26) connected to the free end (25a) of the pin (25); the pusher disc (26) defining a support surface for the external element (11) which is coupled to the valve (10).

9. The respiratory device according to claim 8, **characterised in that** the opening/closing means (22) comprises, on the first level (14), a nail (23) designed to close the secondary pathway (21) when the automatic valve (10) is in the first operating position, so as to allow the flow of gases only along the primary pathway (20).

10. The respiratory device according to claim 9, **characterised in that** the second level (15) has at least one wall (24) which extends at right angles to the septum (16) and which has a transversal channel (C) for the passage of the flow of gases and nebulised medication through the secondary pathway (21) when the valve (10) is in the second operating position.

11. The respiratory device according to claim 10, **characterised in that** when the automatic valve (10) is in the second operating position, the septum (16) closes the primary pathway (20) to enable the passage of the gases mixed with the nebulised medication along the secondary pathway (21).

12. The respiratory device according to claim 11, **characterised in that**, between the septum (16) and the pusher disc (26), it comprises opposing means (12) that tends to return the valve (10) from the second operating position to the first operating position.

13. The respiratory device according to claim 8, **characterised in that** the opening/closing means (22) comprises, on the first level (14), at least one wall (24) designed to close the secondary pathway (21) allowing the flow of gases through the primary pathway (20) only when the valve (10) is in its first operating position.

14. The respiratory device according to claim 13, **characterised in that** the opening/closing means (22) comprises, on the first level (14), two walls (24) between which there is a transversal channel (D) for the passage of the flow of gases through the primary pathway (20) when the valve (10) is in the first operating position; one of the walls (24) being designed to close the secondary pathway (21) when the valve (10) is in its first operating position.

15. The respiratory device according to claim 13 or 14, **characterised in that** the opening/closing means (22) comprises, on the second level (15), a nail (23) designed to close the primary pathway (20) when the automatic valve (10) is in the second operating position so as to enable the passage of gases mixed with nebulised medication along the secondary pathway (21).

16. The respiratory device according to claim 15, **characterised in that** it comprises opposing means (12) which tends to return the valve (10) from the second operating position to the first operating position, said means (12) being positioned between the septum (16) and a bottom wall (28) of a space (V) housing the valve (10), on the side of the first level (14).

17. The respiratory device according to any of claims 1 to 16, **characterised in that** said conditioning member (40) comprises a heat and moisture exchanger element (4).

18. The respiratory device according to any of claims 1 to 16, **characterised in that** said conditioning member (40) comprises a filtering element (5).

19. The respiratory device according to any of claims 1 to 16, **characterised in that** said conditioning member (40) comprises both a heat and moisture exchanger element (4) and a filtering element (5).

20. A ventilator system **characterized in that** it comprises a respiratory device as set out in any of claims 1 to 19.

## Patentansprüche

1. Atemgerät zur Atemgaskonditionierung, umfassend:
eine Hauptbahn (20) und
ein Gehäuse (3), das mit der Hauptbahn (20) in Fluidverbindung gesetzt ist und ein Konditionierungsteil (40) enthält,
eine Nebenbahn (21) zur Abgabe von Arzneimitteln, die unter Bezugnahme auf eine Strömungsrichtung (F) von Gasen vor und nach dem Gehäuse (3) mit der Hauptbahn (20) verbunden ist,
ein automatisches Ventil (10), das zu einer selektiven Aktivierung der Nebenbahn (21) fähig ist und dabei die Hauptbahn (20) ausschließt; **dadurch gekennzeichnet,**
**dass** das Ventil (10) verschiebbar zwischen einer ersten Betriebsposition, in der nur die Hauptbahn (20) aktiv ist, und einer zweiten Betriebsposition, in der nur die Nebenbahn (21) aktiv ist, beweglich ist; wobei das automatische Ventil (10) durch eine Schubwirkung aktiviert wird, wenn ein externes Element (11) damit verkuppelt ist, sodass es sich in die zweite Betriebsposition bewegt, und durch Entfernen des externen Elements (11) deaktiviert wird, sodass es in die erste Betriebsposition zurückkehrt.

2. Atemgerät nach Anspruch 1, wobei die Haupt- und Nebenbahn (20, 21) einen gemeinsamen Einlasskanal (A) aufweisen, **dadurch gekennzeichnet, dass** das automatische Ventil (10) am Einlasskanal (A) positioniert ist.

3. Atemgerät nach Anspruch 1 oder 2, **dadurch gekennzeichnet, dass** es Gegenmittel (12) aufweist, die dazu konzipiert sind, das Ventil in der ersten Betriebsposition zu halten oder dahin zurückzuführen.

4. Atemgerät nach Anspruch 3, **dadurch gekennzeichnet, dass** die Gegenmittel (12) eine Schraubenfeder aufweisen, wobei die Feder durch das Ventil (10) gespannt und belastet wird, wenn das externe Element (11) mit dem Ventil (10) verkuppelt ist, das dabei in die zweite Betriebsposition geschoben wird und in der Ruheposition ausgedehnt ist, wenn das Ventil (10) in der ersten Betriebsposition ist.

5. Atemgerät nach einem der Ansprüche 1 bis 4, **dadurch gekennzeichnet, dass** das automatische Ventil (10) ein Zweiwegeventil ist und einen Hauptkörper (13) umfasst, der durch eine zentrale Trennwand (16) in eine erste Ebene (14) und eine zweite Ebene (15) unterteilt ist.

6. Atemgerät nach Anspruch 5, **dadurch gekennzeichnet, dass** die erste Ebene (14) und die zweite Ebene (15) Öffnungs-/Schließmittel (22) umfassen, welche die Gase durch die Hauptbahn (20) auf der ersten Ebene (14) und die mit einem zerstäubten Arzneimittel gemischten Gase durch die Nebenbahn (21) auf der zweiten Ebene (15) strömen lassen, wodurch jeweils abwechselnd die Nebenbahn (21) und die Hauptbahn (20) geschlossen werden.

7. Atemgerät nach Anspruch 5 oder 6, **dadurch gekennzeichnet, dass** der Hauptkörper (13) einen Stift (25) umfasst, der sich axial und zentral von der Trennwand (16) aus zur zweiten Ebene (15) hin erstreckt.

8. Atemgerät nach Anspruch 7, **dadurch gekennzeichnet, dass** der Hauptkörper (13) eine Druckscheibe (26) umfasst, die mit dem freien Ende (25a) des Stifts (25) verbunden ist, wobei die Druckscheibe (26) eine Stützfläche für das externe Element (11) definiert, das mit dem Ventil (10) verkuppelt ist.

9. Atemgerät nach Anspruch 8, **dadurch gekennzeichnet, dass** die Öffnungs-/Schließmittel (22) auf der ersten Ebene (14) einen Nagel (23) umfassen, der dazu konzipiert ist, die Nebenbahn (21) zu schließen, wenn das automatische Ventil (10) in der ersten Betriebsposition ist, sodass ermöglicht wird, dass die Gase nur entlang der Hauptbahn (20) strömen.

10. Atemgerät nach Anspruch 9, **dadurch gekennzeichnet, dass** die zweite Ebene (15) mindestens eine Wand (24) aufweist, die sich rechtwinklig zur Trennwand (16) erstreckt und einen transversalen Kanal (C) für den Durchtritt von Gasströmungen und zerstäubten Arzneimitteln durch die Nebenbahn (21) aufweist, wenn das Ventil (10) in der zweiten Betriebsposition ist.

11. Atemgerät nach Anspruch 10, **dadurch gekennzeichnet, dass** die Trennwand (16), wenn das automatische Ventil (10) in der zweiten Betriebsposition ist, die Hauptbahn (20) schließt, um den Durchtritt der mit den zerstäubten Arzneimitteln gemischten Gase entlang der Nebenbahn (21) zu ermöglichen.

12. Atemgerät nach Anspruch 11, **dadurch gekennzeichnet, dass** es zwischen der Trennwand (16) und der Druckscheibe (26) Gegenmittel (12) umfasst, die dazu tendieren, das Ventil (10) von der zweiten Betriebsposition in die erste Betriebsposition zurückzuführen.

13. Atemgerät nach Anspruch 8, **dadurch gekennzeichnet, dass** die Öffnungs-/Schließmittel (22) auf der ersten Ebene (14) mindestens eine Wand (24) umfassen, die dazu konzipiert ist, die Nebenbahn (21) zu schließen, wodurch ermöglicht wird, dass die Gase nur dann durch die Hauptbahn (20) strömen, wenn das Ventil (10) in seiner ersten Betriebsposition ist.

14. Atemgerät nach Anspruch 13, **dadurch gekennzeichnet, dass** die Öffnungs-/Schließmittel (22) auf der ersten Ebene (14) zwei Wände (24) umfassen, zwischen denen ein transversaler Kanal (D) für den Durchtritt der Gasströmung durch die Hauptbahn (20) vorhanden ist, wenn das Ventil (10) in der ersten Betriebsposition ist, wobei eine der Wände (24) dazu konzipiert ist, die Nebenbahn (21) zu schließen, wenn das Ventil (10) in seiner ersten Betriebsposition ist.

15. Atemgerät nach Anspruch 13 oder 14, **dadurch gekennzeichnet, dass** die Öffnungs-/Schließmittel (22) auf der zweiten Ebene (15) einen Nagel (23) umfassen, der dazu konzipiert ist, die Hauptbahn (20) zu schließen, wenn das automatische Ventil (10) in der zweiten Betriebsposition ist, sodass der Durchtritt von mit zersträubten Arzneimitteln gemischten Gasen entlang der Nebenbahn (21) ermöglicht wird.

16. Atemgerät nach Anspruch 15, **dadurch gekennzeichnet, dass** es Gegenmittel (12) umfasst, die dazu tendieren, das Ventil (10) von der zweiten Betriebsposition in die erste Betriebsposition zurückzuführen, wobei die Mittel (12) zwischen der Trennwand (16) und einer Bodenwand (28) eines Raumes (V), welcher das Ventil (10) aufnimmt, auf der Seite der ersten Ebene (14) positioniert sind.

17. Atemgerät nach einem der Ansprüche 1 bis 16, **dadurch gekennzeichnet, dass** das Konditionierungsteil (40) ein Hitze- und Feuchtigkeitsaustauschelement (4) umfasst.

18. Atemgerät nach einem der Ansprüche 1 bis 16, **dadurch gekennzeichnet, dass** das Konditionierungsteil (40) ein Filterelement (5) umfasst.

19. Atemgerät nach einem der Ansprüche 1 bis 16, **dadurch gekennzeichnet, dass** das Konditionierungsteil (40) ein Hitze- und Feuchtigkeitsaustauschelement (4) und ein Filterelement (5) umfasst.

20. Belüftungssystem, **dadurch gekennzeichnet, dass** es ein Atemgerät umfasst, das in einem der Ansprüche 1 bis 19 dargelegt wird.

## Revendications

1. Dispositif respiratoire permettant de conditionner des gaz respiratoires, comprenant :
un passage primaire (20) et
un logement (3) placé en communication de fluide avec le passage primaire (20) et contenant un organe de conditionnement (40),
un passage secondaire (21) pour l'administration de médicaments relié au passage primaire (20) tous deux situés en amont et en aval du logement (3) en référence à une direction (F) de l'écoulement des gaz, une vanne automatique (10) capable d'activer sélectivement le passage secondaire (21) et d'exclure le passage primaire (20) ; **caractérisé en ce que** ladite vanne (10) est mobile de façon coulissante entre une première position opérationnelle dans laquelle seul le passage primaire (20) est actif et une seconde position opérationnelle dans laquelle seul le passage secondaire (21) est actif ; la vanne automatique (10) étant activée par une action de poussée lorsque un élément externe (11) lui est associé, de sorte à ce qu'elle se déplace dans la seconde position opérationnelle, et à ce qu'elle soit désactivée par le retrait de l'élément externe (11) de sorte à ce qu'elle retourne dans la première position opérationnelle.

2. Dispositif respiratoire selon la revendication 1, dans lequel lesdits passages primaire et secondaire (20, 21) comportent un canal d'entrée commun (A) **caractérisé en ce que** la vanne automatique (10) est positionnée en correspondance avec ledit canal d'entrée (A).

3. Dispositif respiratoire selon l'une quelconques des revendications 1 ou 2, **caractérisé en ce qu'**il comprend des moyens en opposition (12) conçus pour maintenir ou faire revenir la vanne (10) dans la première position opérationnelle.

4. Dispositif respiratoire selon la revendication 3, **caractérisé en ce que** les moyens en opposition (12) comprennent un ressort hélicoïdal, ledit ressort étant compressé et armé par la vanne (10) lorsque l'élément externe (11) est associé à la vanne (10) qui est ainsi poussé dans la seconde position opérationnelle et qui est étendu dans une position de repos lorsque la vanne (10) se trouve dans la première position opérationnelle.

5. Dispositif respiratoire selon l'une des revendications 1 à 4, **caractérisé en ce que** la vanne automatique (10) est une vanne à deux voies et comprend un corps principal (13) divisé en un premier niveau (14) et un second niveau (15) par une division centrale (16).

6. Dispositif respiratoire selon la revendication 5, **caractérisé en ce que** le premier niveau (14) et le second niveau (15) comprennent des moyens d'ouverture/fermeture (22) laissant passer les gaz à travers le passage primaire (20) au premier niveau (14) et les gaz mélangés à un médicament atomisé à travers le passage secondaire (21) au second niveau (15), en fermant respectivement et alternativement le passage secondaire (21) et le passage primaire (20).

7. Dispositif respiratoire selon les revendications 5 ou 6, **caractérisé en ce que** ledit corps principal (13) comprend un axe (25) se développant de façon axiale et centrale à partir de la division (16) vers le second niveau (15).

8. Dispositif respiratoire selon la revendication 7, **caractérisé en ce que** le corps principal (13) comprend un disque poussoir (26) relié à l'extrémité libre (25a) de l'axe (25) ; le disque poussoir (26) définissant une surface de support pour l'élément externe (11) qui est associé à la vanne (10).

9. Dispositif respiratoire selon la revendication 8, **caractérisé en ce que** les moyens d'ouverture/fermeture (22) comprennent, au premier niveau (14), un ongle (23) conçu pour fermer le passage secondaire (21) lorsque la vanne automatique (10) se trouve dans la première position opérationnelle, de sorte à permettre l'écoulement des gaz uniquement le long du passage primaire (20).

10. Dispositif respiratoire selon la revendication 9, **caractérisé en ce que** le second niveau (15) comporte au moins une paroi (24) qui se développe avec des angles droit par rapport à la division (16) et qui comporte un canal transversal (C) pour permettre le passage de l'écoulement des gaz et du médicament atomisé à travers le passage secondaire (21) lorsque la vanne (10) se trouve dans la seconde position opérationnelle.

11. Dispositif respiratoire selon la revendication 10, **caractérisé en ce que** lorsque la vanne automatique (10) se trouve dans la seconde position opérationnelle, la division (16) ferme le passage primaire (20) pour permettre le passage des gaz mélangés au médicament atomisé le long du passage secondaire (21).

12. Dispositif respiratoire selon la revendication 11, **caractérisé en ce qu'**entre la division (16) et le disque poussoir (26), il comprend des moyens en opposition (12) qui tendent à faire revenir la vanne (10) de la seconde position opérationnelle à la première position opérationnelle.

13. Dispositif respiratoire selon la revendication 8, **caractérisé en ce que** les moyens d'ouverture/fermeture (22) comprennent, au premier niveau (14), au moins une paroi (24) conçue pour fermer le passage secondaire (21) permettant à l'écoulement des gaz de passer à travers le passage primaire (20) uniquement lorsque la vanne (10) se trouve dans sa première position opérationnelle.

14. Dispositif respiratoire selon la revendication 13, **caractérisé en ce que** les moyens d'ouverture/fermeture (22) comprennent, au premier niveau (14), deux parois (24) entre lesquelles se trouve un canal transversal (D) pour permettre le passage de l'écoulement des gaz à travers le passage primaire (20) lorsque la vanne (10) se trouve dans la première position opérationnelle ; l'une des parois (24) étant conçue pour fermer le passage secondaire (21) lorsque la vanne (10) se trouve dans sa première position opérationnelle.

15. Dispositif respiratoire selon les revendications 13 ou 14, **caractérisé en ce que** les moyens d'ouverture/fermeture (22) comprennent au second niveau (15) un ongle (23), conçu pour fermer le passage primaire (20) lorsque la vanne automatique (10) se trouve dans la seconde position opérationnelle, de sorte à permettre le passage des gaz mélangés au médicament atomisé le long du passage secondaire (21).

16. Dispositif respiratoire selon la revendication 15, **caractérisé en ce qu'**il comprend des moyens en opposition (12) qui tendent à faire revenir la vanne (10) de la seconde position opérationnelle à la première position opérationnelle, lesdits moyens (12) étant positionnés entre la division (16) et une paroi inférieure (28) d'un espace (V) logeant la vanne (10), sur le côté du premier niveau (14).

17. Dispositif respiratoire selon l'une quelconque des revendications 1 à 16, **caractérisé en ce que** ledit organe de conditionnement (40) comprend un élément échangeur de chaleur et d'humidité (4).

18. Dispositif respiratoire selon l'une quelconque des revendications 1 à 16, **caractérisé en ce que** ledit organe de conditionnement (40) comprend un élément de filtrage (5).

19. Dispositif respiratoire selon l'une quelconque des revendications 1 à 16, **caractérisé en ce que** ledit organe de conditionnement (40) comprend à la fois un élément échangeur de chaleur et d'humidité (4) et un élément de filtrage (5).

20. Système de ventilation **caractérisé en ce qu'**il comprend un dispositif respiratoire selon l'une des revendications 1 à 19.
